# EUROPEAN PATENT APPLICATION

(11) **EP 2 223 609 A1**
(43) Date of publication of application: **01.09.2010**
(21) Application number: 10160850.3
(22) Date of filing: 24.02.2005
(51) Int. Cl.: A23K 1/00, C12N 1/04, C12N 1/00, C12N 1/20

(54) **Frozen lab culture of individual frozen pellets**

(30) Priority: 24.02.2004 EP 04100714
(62) Divisional of application: 05706788.6
(71) Applicant: Chr. Hansen A/S, 2970 Hørsholm (DK)
(72) Inventor: Knap, Inge, 2700 Broenshoej (DK); Stavnsbjerg, Rikke, 2860 Soeborg (DK); Bisgaard-Frantzen, Hans, 2610 Roedovre (DK)

(57) **Abstract**

A frozen lactic acid bacteria (LAB) culture in a commercial relevant package that has a weight of at least 50 g frozen material, wherein the frozen material is present in the form of individual pellets characterized by that when stored at -50°C for 7 days the individual pellets of the frozen culture are not sticking together and therefore substantially remain as individual pellets.

## Description

### FIELD OF THE INVENTION:

The present invention relates to a frozen lactic acid bacteria (LAB) culture in a commercial relevant package that has a weight of at least 50 g frozen material, wherein the frozen material is present in the form of individual pellets characterized by that when stored at -50°C for 7 days the individual pellets of the frozen culture are not sticking together and therefore substantially remain as individual pellets.

### BACKGROUND OF THE INVENTION:

Microorganisms are involved in the manufacture of food and feed products including most dairy products. Thus, bacterial cultures, in particular cultures of bacteria that are generally classified as lactic acid bacteria are essential in the making of all fermented milk products, cheese and butter. Cultures of such bacteria may be referred to as starter cultures and they impart specific features to various dairy products by performing a number of functions.

Commercial dairy starter cultures are generally composed of lactic acid and citric acid fermenting lactic acid bacteria. In the present context, the expression "lactic acid bacteria" designates a group of Gram positive, catalase negative, non-motile, microaerophilic or anaerobic bacteria which ferment sugar with the production of acids including lactic acid as the predominantly produced acid, acetic acid, formic acid and propionic acid. The industrially most useful lactic acid bacteria are found among *Lactococcus* species, *Streptococcus* species, *Enterococcus* species, *Lactobacillus* species, *Leuconostoc* species and *Pediococcus* species.

Commonly used dairy starter culture strains of lactic acid bacteria are generally divided into mesophilic organisms having optimum growth temperatures at about 30°C and thermophilic organisms having optimum growth temperatures in the range of about 40 to about 45°C. Typical organisms belonging to the mesophilic group include *Lactococcus lactis* subsp. *lactis, Lactococcus lactis* subsp. *cremoris, Leuconostoc mesenteroides* subsp. *cremoris, Pediococcus pentosaceus, Lactococcus lactis* subsp. *lactis* biovar. *diacetylactis* and *Lactobacillus casei* subsp. *casei.* Thermophilic lactic acid bacterial species include as examples *Streptococcus thermophilus, Enterococcusfaecium, Lactobacillus lactis, Lactobacillus helveticus, Lactobacillus delbrueckii* subsp. *bulgaricus* and *Lactobacillus acidophilus.*

The dairy starter cultures are also classified according to their specific species composition and preferred industrial use. A pure starter culture comprises only a single specie and a mixed culture comprises two or more different species. Commercial relevant mesophilic mixed cultures include:
*"O-culture"* comprising *Lactococcus lactis* subsp. *lactis* and *Lactococcus lactis* subsp. *cremoris.*
*"D-culture"* comprising *Lactococcus lactis* subsp. *lactis, Lactococcus lactis* subsp. *cremoris* and *Lactococcus lactis* subsp. *lactis* biovar. *diacetylactis.*
*"L-culture"* comprising *Lactococcus lactis* subsp. *lactis, Lactococcus lactis* subsp. *cremoris* and *Leuconostoc mesenteroides* subsp. *cremoris.*
*"LD-culture"* comprising *Lactococcus lactis* subsp. *lactis, Lactococcus lactis* subsp. *cremoris, Lactococcus lactis* subsp. *lactis* biovar. *diacetylactis* and *Leuconostoc mesenteroides* subsp. *cremoris.*

An *O-culture* is used to make cheese without holes (Cheddar, Cheshire, Feta). A *D-culture* is used to make butter. A *L-culture* is used to cheese with only small holes (cottage cheese) and curdled milk products with low CO₂-production. A *LD-culture* is used to make cheese with normal hole sizes, curdled milk products (junket) and sour butter. Commercially, a LD-culture is currently one of the most used mixed cultures.

Commercial relevant thermophilic mixed cultures include:
*"Yoghurt culture"* comprising *Streptococcus thermophilus* and *Lactobacillus delbrueckii* subsp. *bulgaricus.*
*"Thermophil cheese culture"* comprising *Streptococcus thermophilus* and *Lactobacillus helveticus.*

An *Yoghurt culture* is used to make yoghurt and special Italian cheeses. An *Thermophil cheese culture* is used to make emmentaler cheese and special Italian cheeses.

Commercial starter cultures may commonly be distributed as frozen cultures. Highly concentrated frozen cultures are commercially very interesting since such cultures can be inoculated directly into milk without intermediate transfer. In others words, such highly concentrated frozen cultures comprises so many bacteria that dairies do not have to make in-house bulk starters. A "bulk starter" is defined herein as a starter culture propagated at the dairy plant for inoculation into milk. Highly concentrated cultures may be referred to as direct vat set (DVS)-cultures.

In order to comprise sufficient bacteria a commercial relevant highly concentrated frozen culture generally has a weight of at least 50 g and a content of viable bacteria of at least 10⁹ colony forming units (CFU) per g.

Another presentation of commercial highly concentrated DVS-starter cultures is as freeze-dried or lyophilized cultures in the form of a powder. In this form, the starter can be shipped without refrigeration.

The article of F.J. Chavarri et al (Biotechnology letters, vol 10, 1, 11- 16 (1988), "Cryoprotective agents for frozen concentrated starters from non-bitter Streptococcus Lactis strains") describes that the storage viability of a frozen pure *Streptococcus lactis* culture could be improved by addition of 5% lactose or 5% sucrose. The lactose or sucrose worked as cryoprotective agents. *Streptococcus lactis* is a former name of *Lactococcus lactis* subsp. *lactis.*

Similarly, the article of R. Cárcoba et al (Eur Food Res Technol (2000) 211, 433 - 437, "Influence of cryoprotectants on the viability and acidifying activity of frozen and freeze-dried cells of the novel starter strain Lactococcus lactis subsp. lactis CECT 5180") describes that the storage viability of a frozen pure *Lactococcus lactis* subsp. *lactis* culture could be improved by addition of different cryoprotective agents such as sugars (lactose, sucrose and trehalose), glutamic acid and gelatin.

The present inventors are not aware of any commercial available highly concentrated frozen cultures that comprise significant amounts of cryoprotective agents.

EP259739 describes different suitable cryoprotective agents for freeze-dried cultures. A freeze-dried culture in the form of a powder is physically significant different from a frozen culture among others due to that a freeze-dried powder comprises significant less water as compared to a frozen culture. Accordingly, it is submitted that the skilled person would prima facie not consider that a specific cryoprotective agent described as useful for a freeze-dried culture would also be similar useful in a frozen culture.

WO00/39281 (Chr. Hansen A/S) describes a liquid starter culture stabilized by different cryoprotective agents. Page 5, lines 5-7 reads "the expression "liquid starter culture" relates to non-frozen liquid starter cultures having a liquid phase, e. g. an aqueous phase, content that is typically in the range of 50-90% by weight". Consequently, the liquid culture described in WO00/39281 is a non-frozen culture.

### SUMMARY OF THE INVENTION:

Commercially, a frozen lactic acid bacteria (LAB) culture is normally provided in a suitable package (e.g. in a 2 L tetra pack of carton). It is normally stored at a storage temperature of around -50°C and the frozen material is present in the form of individual pellets of a relatively small weight.

Prior to the present invention, the present inventors believed that there were no significant problems with respect to storage of such commercial relevant frozen lactic acid bacteria (LAB) cultures and as said above, the present inventors are not aware of any commercial available highly concentrated frozen cultures that comprise significant amounts of additive compounds such as cryoprotective agents.

However, based on different studies the present inventors identified that when a number of commercial relevant cultures where stored at -50°C for 7 days or longer the individual pellets were sticking together and making larger clumps. Commercially this gives problems due to e.g. that it is must more difficult to administer an adequate dose from the culture package and sometimes it is even difficult to get the clumped culture out of the package in a suitable way.

Further studies identified that the "problematic" cultures could be characterized by having a Tm' value (onset temperature of initial ice melting) of the frozen lactic acid bacteria (LAB) culture below the storage temperature of -50°C. The Tm' value is a standard known term in the food industry and is routinely measured by DSC techniques. It relates to the onset temperature of initial ice melting of the food product (here the frozen LAB culture). For further details reference are made to the textbooks "Food Chemistry" (ISBN 0-8247-9346-3) and "Phase Transition in Foods" (ISBN 0-12-595340-2).

Without being limited to theory, it could be that when a frozen culture has a Tm' value below the storage temperature of -50°C a small amount of free water is released during storage and this released water could then maybe cause that the individual pellets stick together and form larger clumps.

In summary, the work of the present inventors has identified hitherto unrecognized storage problems in relation to some types of commercial relevant highly concentrated frozen lactic acid bacteria cultures. Once having identified this problem, the present inventors could start to try to solve the problem.

Independent of any theoretical possible explanation, the present inventors identified that by adding certain known relevant additive compounds to a problematic frozen culture one could get a frozen culture which after 7 days storage at -50°C did not form clumps of individual pellets. Said in another way, the individual pellets of the frozen culture were not sticking together and therefore substantially remain as individual pellets.

Overall, the relevant additive compounds may be characterized by that they are able to increase the Tm' value, of the frozen culture, to a value above the -50°C such as to a Tm' value from -49°C to -15°C.

The working examples herein describe preferred examples of suitable additive compounds. Described compounds include trehalose, maltodextrin, cyclodextrin, spray gum, Fish gelatin bloom and maltitol. Based on common general knowledge the skilled person is perfectly capable of identifying further relevant additive compounds that are able to increase the Tm' value, of the frozen culture, to a value above the -50°C.

As said above, in order to comprise sufficient bacteria a commercial relevant highly concentrated frozen culture generally has a weight of at least 50 g and a content of viable bacteria of at least 10⁹ colony forming units (CFU) per g. The pure *Lactococcus lactis* subsp. *lactis* cultures described in the articles of F.J. Chavarri et al and R. Carboba et al (see above) are in the present context not considered commercial relevant highly concentrated frozen cultures since they are made on must smaller scale and comprises significant less grams of frozen culture.

Accordingly, a first aspect of the invention relates to a frozen lactic acid bacteria (LAB) culture in a commercial relevant package that has a weight of at least 50 g frozen material, wherein the frozen material is present in the form of individual pellets, a content of viable bacteria of at least 10⁹ colony forming units (CFU) per g frozen material and comprises from 0.5% to 12% of an additive compound measured as w/w of the frozen material, wherein the additive compound is an additive compound that is selected from the group of compounds that,
by using an amount of 10% of the additive compound measured as w/w of the frozen material, are able to increase the Tm' (onset temperature of ice melting) of the frozen lactic acid bacteria (LAB) culture, which without the additive compound has a Tm' value from -65°C to -50°C, to a Tm' value from -49°C to -15°C (measured by DSC)
and wherein the frozen lactic acid bacteria (LAB) culture is characterized by that when stored at -50°C for 7 days the individual pellets of the frozen culture are not sticking together and therefore substantially remain as individual pellets, where this is measured by following test
the individual pellets of the frozen culture are pellet frozen in liquid nitrogen and 100 individual pellets (around 5 - 100 g of pellets) are poured into a petridish, thus forming a thin layer of loose individual single pellets, placed at -50°C for 7 days of storage and examined to see if the pellets are still loose or if the pellets had made clumps or are sticking together wherein the criteria for that the individual pellets of the frozen culture substantially remain as individual pellets are that at least 80 of the 100 individual pellets remain as loose individual single pellets;
with the exception of a frozen lactic acid bacteria (LAB) culture that comprises LAB that are that are able to utilize sucrose and wherein the culture comprises a cryoprotective agent compound selected from the group consisting of sucrose in an amount from 2 % to 12 % of sucrose measured as w/w of the frozen material; and trehalose in an amount from 4 % to 6 % of trehalose measured as w/w of the frozen material.

The "disclaimer" described at the end of the first aspect relates to the PCT application with the application number PCT/DK2004/000025. It was filed 19 of January 2004. At the filing date of the present application the PCT application PCT/DK2004/000025 was not published.

PCT application PCT/DK2004/000025 relates to improving storage stability of a frozen culture. It does not describe the "pellet sticking" problem of the present invention. The general main claim 1 relates to "a frozen lactic acid bacteria (LAB) culture that comprises LAB that are that are able to utilize sucrose, has a weight of at least 50 g frozen material and a content of viable bacteria of at least 10⁹ colony forming units (CFU) per g frozen material, **characterized in that** the frozen culture comprises from 0.5% to 80% of a cryoprotective agent measured as w/w of the frozen material."

As suitable cryoprotective agents and used in the general amount mentioned are, in the PCT application, described a number of compounds. Directly and unambiguously described specific examples include:
Example 1: Frozen LD-cultures of F1 DaN and CH N11 comprising 6%, 10%, 22% or 36% sucrose as stabilizing cryoprotective agent;
Example 2: Frozen LD-culture of F1 DaN, CH N11 & CH N19 comprising 5% Trehalose and cultures with 3, 5, 6, 8, 9, 10 or 13% of sucrose; and
Example 4: Frozen Lactobacilus acidophislus (La-5) comprising 32% of sucrose and a mixture of 16% maltodextrine and 16% sucrose.
All the LAB of the PCT application examples are able to utilize sucrose.

With respect to the frozen culture of the present invention, the additive compound should preferably be added to the viable bacteria before they are frozen.

Accordingly, in a second aspect the invention relates to a method for making a frozen lactic acid bacteria (LAB) culture of the first aspect of the invention and embodiments as described herein comprising following steps:
(i) adding an additive compound to viable bacteria to get at least 50 g of material with a content of viable bacteria of at least 10⁹ colony forming units (CFU) per g material and comprising the additive compound in an amount from 0.5% to 12% measured as w/w of the material,
(ii) freezing the material to get frozen material, and
(iii) packing the frozen material in a suitable way to get a packed frozen lactic acid bacteria (LAB) culture of the first aspect of the invention and embodiments as described herein.

A third aspect of the invention relates to a frozen lactic acid bacteria (LAB) culture obtainable by the method for making a frozen lactic acid bacteria (LAB) culture of the second aspects of the invention.

A fourth aspect of the invention relates to use of the frozen lactic acid bacteria (LAB) culture as described above in a process for making a food or feed product.

### DEFINITIONS:

Prior to a discussion of the detailed embodiments of the invention is provided a definition of specific terms related to the main aspects of the invention.

The term "LAB that are that are able to utilize sucrose" denotes LAB that are able to ferment the sugar sucrose with the production of acids. This is the same definition as in PCT application number PCT/DK2004/000025.

The term "material" of the culture denotes the relevant substances of the culture including both the viable bacteria and cryoprotective agent. Possible packing is not included. Consequently, the weight of the material of the culture is not including the weight of possible packing.

The term "packing" or "package" should be understood broadly. It denotes that the frozen lactic acid bacteria (LAB) culture should be packed in order could to be provided to the user. It may be packed in a bottle, a tetra-pack, etc.

The term "an additive compound" may in the present context be a single specific additive compound or it may be two or more different additive compounds. Accordingly, the w/w percentage of the additive compound(s) within the culture material should be understood as the sum of the amount of additive compound(s). Preferably, the term relates to a compound that is added to the culture after fermentation. Accordingly, it may be a compound that is not present in a significant amount in the culture fermentation broth as such.

Embodiments of the present invention is described below, by way of examples only

### DRAWINGS

Figure 1: DSC Phase transition curves. For further details see working Example 2.
Figure 2: DSC phase transition curves were the Tm onset and Tg onset were found. The correlation between Tm and amount of disaccarides added can be seen in this figure. For further details see working Example 3.

### DETAILED DESCRIPTION OF THE INVENTION:

### Tm' value

As explained above, the Tm' value is a standard known term in the food industry and is routinely measured by DSC techniques. It relates to the onset temperature of initial ice melting of the food product (here the frozen LAB culture).

Preferably, the Tm' value is measured by use of the DSC protocol described in the section named "Measurement of Tm' and Tg" of working example 1 herein.

### Pellet clumping test

As explained with respect to the first aspect of the invention the test to analyze if the frozen lactic acid bacteria (LAB) culture is a culture that may be characterized by that when stored at - 50°C for 7 days the individual pellets of the frozen culture are not sticking together and therefore substantially remain as individual pellets is a test comprising following:
the individual pellets of the frozen culture are pellet frozen in liquid nitrogen and 100 individual pellets (around 5 - 100 g of pellets) are poured into a petridish, thus forming a thin layer of loose individual single pellets, placed at -50°C for 7 days of storage and examined to see if the pellets are still loose or if the pellets had made clumps or are sticking together wherein the criteria for that the individual pellets of the frozen culture substantially remain as individual pellets are that at least 80 of the 100 individual pellets remain as loose individual single pellets. More preferably at least 90 of the 100 individual pellets remain as loose individual single pellets and even more preferably at least 95 of the 100 individual pellets remain as loose individual single pellets.

To examine and count individual pellets that remain as loose individual single pellets may be done visually. It is within the skilled person capacity to do this in a consistent way where the results would, within normal limited technical uncertainty, be consistent and repeatable. Working example 1 herein provides further technical details.

### A frozen lactic acid bacteria (LAB) culture

Preferably, the term "a frozen lactic acid bacteria (LAB) culture" denotes herein a culture which without comprising the added additive compound as described herein has a Tm' value of from -65°C to -50°C.

The LAB of the culture may be any in particular commercial relevant LAB.

Preferably, the LAB is a LAB selected from the group comprising *Bifidobacterium* spp., *Brevibacterium* spp., *Propionibacterium* spp., *Lactococcus* spp. including *Lactococcus lactis* subsp. *lactis* and *Lactococcus lactis* subsp. *cremoris, Lactobacillus* spp. including *Lactobacillus acidophilus, Streptococcus* spp., *Enterococcus* spp., *Pediococcus* spp., *Leuconostoc* spp., *Oenococcus* spp. and fungal spp. including *Pencillium* spp., *Cryptococcus* spp., *Debraryomyces* spp., *Klyveromyces* spp. and *Saccharomyces* spp.

The industrially most useful lactic acid bacteria are found among *Lactococcus* species, *Streptococcus* species, *Enterococcus* species, *Lactobacillus* species, *Leuconostoc* species and *Pediococcus* species.

The term "mixed lactic acid bacteria (LAB) culture" denotes a mixed culture that comprises two or more different LAB species. The term a "pure lactic acid bacteria (LAB) culture" denotes a pure culture that comprises only a single LAB species specie.

The culture as described herein may be a mesophilic culture consisting of mesophilic bacteria having optimum growth temperatures at about 30°C.

Typical organisms belonging to the mesophilic group include *Lactococcus lactis* subsp. *lactis, Lactococcus lactis* subsp. *cremoris, Leuconostoc mesenteroides* subsp. *cremoris, Pediococcus pentosaceus, Lactococcus lactis* subsp. *lactis* biovar. *diacetylactis* and *Lactobacillus casei* subsp. *casei.* Thermophilic lactic acid bacterial species include as examples *Streptococcus thermophilus, Enterococcus faecium, Lactobacillus lactis, Lactobacillus helveticus, Lactobacillus delbrueckii* subsp. *bulgaricus* and *Lactobacillus acidophilus.*

The culture as described herein may comprise LAB that are able to utilize sucrose. The *Leuconostoc mesenteroides* subsp. *cremoris* is able to utilize sucrose. Among others, it is present in *a L-culture* and a *LD-culture.*

Consequently, in a preferred embodiment the frozen culture is a *L-culture* or more preferably a *LD-culture. A L-culture* and a *LD-culture* are examples of mesophilic cultures. Further they are mixed cultures. Consequently, a culture as described herein is preferably a mixed culture, more preferably a mesophilic mixed culture.

*A L-culture* comprises *Lactococcus lactis* subsp. *lactis, Lactococcus lactis* subsp. *cremoris* and *Leuconostoc mesenteroides* subsp. *cremoris.*

*A LD-culture* comprises *Lactococcus lactis* subsp. *lactis, Lactococcus lactis* subsp. *cremoris, Lactococcus lactis* subsp. *lactis* biovar. *diacetylactis* and *Leuconostoc mesenteroides* subsp. *cremoris.*

The specific amount of the individual bacterial species may vary in accordance with the specific required use. The skilled person is aware of this and capable of determining the preferred mixed culture composition according to the required needs.

For instance, if aroma is required a relatively high percentage of the aroma making bacteria *Lactococcus lactis* subsp. *lactis* biovar. *diacetylactis* and *Leuconostoc mesenteroides* subsp. *cremoris* could be preferred.

A preferred LD-culture comprises:

| | |
|---|---|
| *Lactococcus lactis* subsp. *lactis,* *Lactococcus lactis* subsp. *cremoris* | 60 - 95 %, preferably 70 - 90% |
| *Lactococcus lactis* subsp. *lactis* biovar. *diacetylactis,* *Leuconostoc mesenteroides* subsp. *cremoris* | 5 - 40 %, preferably 10 to 30% |

Within the ranges above, it is preferred to have from 0.25 to 6% *of Leuconostoc mesenteroides* subsp. *cremoris* and from 7 to 30% of *Lactococcus lactis* subsp. *lactis* biovar. *diacetylactis.*

Of course the total percentage sum of the 4 different LAB specifies cannot exceed 100%. However, it may be less than 100% if other bacteria than the 4 mentioned ones are present in the *LD-culture.* Working examples 1 and 2 herein provides examples of stabilized *LD-cultures.*

The culture as described herein may be a thermophilic culture consisting of thermophilic bacteria having optimum growth temperatures in the range of about 40 to about 45°C.

The culture as described herein may comprise LAB that are able to utilize sucrose. The thermophilic *Lactobacillus acidophilus* is able to utilize sucrose. Accordingly, in a preferred embodiment the frozen culture is a culture comprising *Lactobacillus acidophilus,* preferably a pure *Lactobacillus acidophilus* culture.
The thermophilic *Streptococcus thermophilus* is able to utilize sucrose. In a preferred embodiment the frozen culture is a mixed
*"Yoghurt culture"* comprising *Streptococcus thermophilus* and *Lactobacillus delbrueckii* subsp. *bulgaricus;* or
*"Thermophil cheese culture"* comprising *Streptococcus thermophilus* and *Lactobacillus helveticus.*

The culture as described herein may comprise LAB that are not able to utilize sucrose. The *O-culture* does not comprise bacteria that are able to utilize sucrose. An *O-culture* comprises *Lactococcus lactis* subsp. *lactis* and *Lactococcus lactis* subsp. *Cremoris.*

### Highly concentrated frozen lactic acid bacteria cultures

The frozen cultures as described herein are what in the food industry may be termed highly concentrated frozen lactic acid bacteria cultures. In order to comprise sufficient bacteria such cultures should be relatively big (have a sufficient weight) combined with a relatively high concentration of viable bacteria. It is obvious that if relatively more bacteria is required the weight and/or the concentration of viable bacteria should be increased.

Preferably, a frozen lactic acid bacteria (LAB) culture as described herein has a weight of at least 100 g frozen material, more preferably a weight of at least 250 g frozen material, even more preferably a weight of at least 500 g frozen material and most preferably a weight of at least 900 g frozen material. Preferably, the weight of the frozen material is less than 500 kg.

Preferably, a frozen lactic acid bacteria (LAB) culture as described herein has a content of viable bacteria of at least 5x10⁹ colony forming units (CFU) per g frozen material, more preferably a content of viable bacteria of at least 10¹⁰ colony forming units (CFU) per g frozen material, and most preferably a content of viable bacteria of at least 2x10¹⁰ colony forming units (CFU) per g frozen material.

Fermentation and suitable fermentations media for LAB are known in the art and the skilled person is capable of selecting a suitable media and fermentation conditions in relation to the specific LAB. Suitable media and fermentations are given in the working example section herein.

In order to get sufficient amount of bacteria, it is in the present context preferred to make a relatively large-scale fermentation in suitable big fermentation tanks. Fermentation tanks of at least 50 1, preferably at least 90 1 or bigger are preferred.

After a suitable fermentation, the viable bacteria are preferably isolated by removal of the liquid (supernatant) of the fermentation media (e.g. by centrifugation). The isolated viable bacteria may be termed the isolated biomass. The isolated viable bacteria shall preferably have a content of viable bacteria of at least 10⁹ colony forming units (CFU) per g or ml.

The frozen culture may be packaged is a suitable way in order to be provided to the user.

### Additive compound

As discussed above, preferably the relevant additive compounds may be characterize by that they are able to increase the Tm' value, of the frozen culture, to a value above the -50°C such as to a Tm' value from -49°C to -15°C, more preferably to a Tm' value from -43°C to -15°C and even more preferably to a Tm' value from -39°C to -15°C.

Working example 2 herein illustrates a rapid experimental strategy to identify relevant additive compounds. To a "model" frozen culture with a Tm' value below -50°C (in example 2 "model" culture has a Tm' value of -54°C) was added different relevant compounds (10% W/W) and the Tm' values before and after addition were measured by DSC.

The "model" culture of example 2 and the test protocol of this example 2 is preferably used to evaluate if specific additive compounds of interest may be characterize by that they are able to increase the Tm' value, of the frozen culture, to a value above the -50°C such as to a Tm' value from -49°C to -15°C, more preferably to a Tm' value from -43°C to -15°C, even more preferably to a Tm' value from -39°C to -15°C.

In working example 2 it can be seen that Cyclodextrin increased Tm' to -44°C, Maltitol increased Tm' to -42°C, Trehalose increased Tm' to -38°C, Fish gelatin increased Tm' to - 37°C, Maltodextrine increased Tm' to -32°C and Spray gum increased Tm' to -31°C.

Preferably, the additive compound is compound with a molecular weight (MW) from 500 to 100000 g/mol, more preferably 750 to 100000 g/mol, even more preferably from 1000 to 40000 g/mol and most preferably from 1500 to 15000 g/mol.

In a preferred embodiment the additive compound is a cryoprotective agent.

The term "a cryoprotective agent" denotes a substance that is able to improve the storage stability of the frozen culture. In the present context it may be a single specific cryoprotective agents or it may be two or more different agents. Accordingly, the w/w percentage of the cryoprotective agent(s) within the culture material should be understood as the sum of the amount of cryoprotective agent(s). A preferred way to determine whether a substance is a cryoprotective agent that is able to improve the storage stability of the frozen culture is to spilt a culture, as described herein, in two samples, add a specified amount of the cryoprotective agent to one of them, freezing both of them and measure the milk acidifying activity of the cultures on the same day as freezing and periodically up to one year under frozen storage. If the culture with cryoprotective agent has improved milk acidifying activity seen over the storage period the substance is a cryoprotective agent. A suitable milk acidifying activity assay is given in working examples herein.

The cryoprotective agent may preferably be selected from proteins or protein hydrolysates. Preferred suitable examples of these include the ones selected from the group consisting of Malt extract, Skimmed milk powder, Whey powder, Yeast extract, Gluten, Collagen, Gelatin, Elastin, Keratin, and Albumins.

More preferably the cryoprotective agent is a carbonhydrate. Preferred suitable examples of these include the ones selected from the group consisting Pentoses (eg. Ribose, Xylose), Hexoses (eg. fructose, mannose, Sorbose), Disaccharides (eg. Sucrose, Trehalose, Melibiose, Lactulose), Oligosaccharides (eg. Raffinose), Oligofrutoses (eg. Actilight, Fribroloses), Polysaccharides (eg. Maltodextrins, Xanthan Gum, Pectin, Alginate, Microcrystalline cellulose, Dextran, PEG), and Sugar alcohols (Sorbitol, Manitol). Most preferably the carbonhydrate is a carbonhydrate with a molecular weight (MW) from 500 to 100000 g/mol, more preferably 750 to 100000 g/mol, even more preferably from 1000 to 40000 g/mol and most preferably from 1500 to 15000 g/mol.

In a very preferred embodiment, the additive compound is an additive compound selected from the group consisting of Cyclodextrin, Maltitol, Fish gelatin, Maltodextrine (preferably maltodextrine 6 to maltodextrine 19) Spray gum and IMP.

Preferably the frozen culture comprises from 1% to 10% of an additive compound measured as w/w of the frozen material, more preferably from 2% to 8% of an additive compound measured as w/w of the frozen material and even more preferably from 3% to 7% of an additive compound measured as w/w of the frozen material.

The addition of the additive compound such as cryoprotective agent to the, after fermentation, isolated viable bacteria (biomass) may be done by mixing solid cryoprotective agent with the biomass for e.g. 30 minutes at a suitable temperature. If the cryoprotective agent is e.g. maltodextrin a suitable temperature may be room temperature. Alternatively a sterile solution of the cryoprotective agent may be mixed with the biomass.

### A method for making a frozen lactic acid bacteria (LAB) culture

As said above, a second aspect the invention relates to a method for making a frozen lactic acid bacteria (LAB) culture of the first aspect of the invention and it embodiments comprising following steps:
(i) adding an additive compound to viable bacteria to get at least 50 g of material with a content of viable bacteria of at least 10⁹ colony forming units (CFU) per g material and comprising the additive compound in an amount from 0.5% to 12% measured as w/w of the material,
(ii) freezing the material to get frozen material, and
(iii) packing the frozen material in a suitable way.

As discussed above, the herein most relevant "problematic" cultures are frozen lactic acid bacteria (LAB) cultures which without comprising the added additive compound as described herein has a Tm' value of from -65°C to -50°C.

Accordingly, in a preferred embodiment before making the addition of the additive compound according to step (i) above one has measured the Tm' value of the frozen lactic acid bacteria (LAB) culture without comprising the additive compound and identified that it has a Tm' value of from -65°C to -50°C.

Alternatively, before making the addition of the additive compound according to step (i) above one has performed a pellet clumping test (see above) and identified that the individual pellets of the frozen culture stick together at storage at -50°C.

Preferably, after the addition of the additive compound is the Tm' value of the frozen lactic acid bacteria (LAB) culture comprising the additive compound measured and it is verified that the Tm' value is from -49°C to -15°C, more preferably from -39°C to -15°C.

Alternatively, after the addition of the additive compound one performs a pellet clumping test (see above) and identifies that at least 80 of the 100 individual pellets remain as loose individual single pellets.

### Use of the frozen lactic acid bacteria (LAB) culture

A frozen lactic acid bacteria (LAB) culture as described herein may be used in a process for making a food or feed product according to the art.

*A L-culture* is preferably used to make cheese with only small holes (cottage cheese) and curdled milk products with low CO₂-production.

*A LD-culture* is preferably used to make cheese with normal hole sizes, curdled milk products (junket) and sour butter.

### EXAMPLES:

### Materials and methods

### Acidifying activity assay and CFU analysis:

Frozen culture was inoculated on a 0.01 % level in 200 ml sterilized reconstituted skimmed milk (RSM) containing 9,5% solid matter and RSM were incubated at 30°C for 6h to permit acidification of the substrate material. The acidification activity was measured as described by Analytical Procedure Q-AM-052, "acidification activity - UHT", Chr. Hansen A/S (Denmark). CFU analysis was measured and calculated as described by analytical Procedure Q-AM-071, "Enumeration of microorganisms" and Q-AM-022 "Calculation of total count, Chr. Hansen A/S (Denmark) using substrate 1209 - LD agar DK-med-rec-123, Chr-Hansen A/S (Denmark) or MRS agar.

### Example 1:

R604-E (commercially available frozen *O-culture,* Chr. Hansen A/S, Denmark) tends to form sticky pellets during frozen storage. In the present study this problem is approached by taking a closer look at the melting temperature, and trying to increase it by adding caseinate, sucrose or malto dextrin.

### Aim:

To evaluate if it is possible to raise the melting point of F-DVS of R604-E by using additives.
The effect of using additives to increase the melting point of R604-E is evaluated:
visually, and
by measuring the Tg' and Tm' by DSC for each of the tested formulations.

### i) Material:

2 kilos of F-DVS R 604-E (Batch 2441258, material 616581).

### Manufacture of F-DVS R604-E:

The fermentation medium had the following composition: Casein peptone, 30 g/l; Primatone, 30 g/l; soy peptone, 30 g/l; yeast peptone, 15 g/l; MgSO₄, 1,5 g/l; Na-ascorbate, 3 g/l; and lactose 50 g/l.
The medium was sterilised by UHT-treatment. The finished medium had a pH of 6,5.

### Fermentation condition for F-DVS R604:

The fermentation was performed in a 100 1 fermentation tank at 30°C, stirred at 50 rpm. 1 % of the culture mentioned above was used as inoculum. The anaerobic fermentation was run with nitrogen in the headspace and a pressure of about 2 bar. The cultures were allowed to acidify to pH 6.2 . The pH was subsequently maintained at 6,2 by controlled addition of 13,4 N NH₄OH. When no further base consumption was detected, the respective culture was cooled down to about 10°C.

Following cooling, the fermentation broth was concentrated by centrifugation and subsequently frozen as pellets in liquid nitrogen. Frozen cultures are normally stored at
-50°C.

### ii) Additives solution used for formulation to raise the melting point:

50% (w/w) sucrose solution.
10% (w/w) Na-caseinate solution.
30% (w/w) Malto Dextrin 10 solution.
30% (w/w) Malto Dextrin 2 solution.

### iii) Recipe for formulation of F-DVS R604-E:

The frozen concentrate was thawed and mixed with additives according to Table 1.

**Table 1. Formulations of R604-E**

| **Formulation ID** | **Amount cell concentrate (g)** | **Additives** **(g)** | **Dilution of conc.** **x** | **Additives*** **(%)** |
|---|---|---|---|---|
| F-DVS R604E/Reference | 300 | 0 | 1 | 0 |
| F-DVS R604E/6% Sucrose | 300 | 41 | 1,14 | 6 |
| F-DVS R604E/10% Sucrose | 300 | 75 | 1,25 | 10 |
| F-DVS R604E/6% Malto Dextrin 10 | 300 | 75 | 1,25 | 6 |
| F-DVS R604E/6% Malto Dextrin 2 | 300 | 75 | 1,25 | 6 |
| F-DVS R604E/2% Na-caseinate | 300 | 75 | 1,25 | 2 |

| | | | | |
|---|---|---|---|---|
| *) g dry matter additive / g concentrate | | | | |

### Visual evaluation of melting point of F-DVS R604.

The 6 formulations of F-DVS R-604 E were pellet frozen in liquid nitrogen and 100 individual pellets (around 20 - 30 g) of pellets were poured into petridishes, thus forming a thin layer of loose single pellets.
One sample of each of the formulations was placed at -50°C. After 7 days of storage the samples were examined to see if they were still loose or if the pellets had made clumps or seemed sticky - and if so - their willingness to be shaken into loose particles again.

**Table 2; Visual inspection of frozen pellets.**

| **Sample marked :** | | **Stored at minus 50°C** |
|---|---|---|
| F-DVS R604-E Reference | Batch 2441258 | - |
| F-DVS R604-E 6% Sucrose | Batch 2441258 | - |
| F-DVS R604-E 8 10% Sucrose | Batch 244125 | ++ |
| F-DVS R604-E 6% Malto Dextrin 10 | Batch 2441258 | +++ |
| F-DVS R604-E 6% Malto Dextrin 2 | Batch 2441258 | +++ |
| F-DVS R604-E 2% Na-caseinate | Batch 2441258 | - |

| | | |
|---|---|---|
| - = Clump, caked or sticky. (less than 20 of the 100 individual pellets remain as loose individual single pellets) + = Partly loose particles. (less than 60 of the 100 individual pellets remain as loose individual single pellets) ++ = Nearly loose particles. (at least 80 of the 100 individual pellets remain as loose individual single pellets) +++ = Loose particles (at least 90 of the 100 individual pellets remain as loose individual single pellets) | | |

### Measurement of Tm' and Tg:

The samples were prepared in 100 ul diglers and frozen in liquid nitrogen. One sample of each of the formulations and F-DVS R604 was placed at -50°C. Another set of samples was placed at -80°C.
The phase transition was measured on a DSC Mettler Toledo 822e 100 ul digler (onset used temperature program, insert temp -90°C, temp. program; 5°C/min. -130°C - 0°C
The glass transition temperature (Tg) and melting point (Tm') were measured periodically up to 1 week in order to observe any annealing reaction taking place during storage. Formulations placed at -80°C are reference samples stored in glass state, thus their Tg and Tm' should be unchanged.

**Table 3. Tm' measurements for samples at frozen storage.**

| | **Stored at minus 50°C/Annealing** | **Stored at minus 50°C/Annealing** | **Stored at minus** | **Stored at minus** |
|---|---|---|---|---|
| | **Day 0** | **Day 6** | **80°C/Day 0** | **80°C/Annealing Day 6** |
| **Sample marked:** | **Tm'** | **Tm'** | **Tm'** | **Tm'** |
| F-DVS R604-E | -57 | -56 | -57 | -54 |
| Batch 2441258 | | | | |
| Reference | | | | |
| F-DVS R604-E | -49 | -49 | -49 | -47 |
| Batch 2441258 | | | | |
| 6% Sucrose | | | | |
| F-DVS R604-E | -47 | -42 | -46 | -44 |
| Batch 2441258 | | | | |
| 10% Sucrose | | | | |
| F-DVS R604-E | -45 | -44 | -44 | -42 |
| Batch 2441258 | | | | |
| 6% Malto Dextrin 10 | | | | |
| F-DVS R604-E | -44 | -42 | -43 | -44 |
| Batch 2441258 | | | | |
| 6% Malto Dextrin 2 | | | | |
| F-DVS R604-E | (-55) | -58 | - | -56 |
| Batch 2441258 | | | | |
| 2% Na-caseinate | | | | |

Use of >6% sucrose and 6% malto dextrin (2 or 10) increase the Tm' value of frozen pellets. It is not possible to see any effect of Na-caseinate. From the visual inspection 10% sucrose and the two different malto dextrin show positive effect against the tendency to make sticky pellets.

### Example 2:

To identify what kind of additives that could increase the melting point of frozen culture a screening study was made. The following additives were tested:
Trehalose, Malto dextrin 12, cyclo dextrin, spray gum, PEG, Fish gelatin, maltitol, Sodium chloride, glycerol.

### i) Material:

F-DVS R 604-E (Batch 2471755, material 616581) for details please see Example1.

### ii) Additives solution used for formulation to raise the melting point:

50% (w/w) trehalose solution.
30% (w/w) Malto Dextrin 12.
30% (w/w) cyclodextrin
30% (w/w) Spray gum
30% (w/w) PEG
30% (w/w) Fish gelatin bloom 0
30% (w/w) maltitol
30% (w/w). Sodium Chloride
30% (w/w) Glycerol

### iii) Recipe for formulation of F-DVS R604-E:

The frozen concentrate was thawed and mixed with the different additives to a final formulation of 10% (W/W).

### Measurement of Tm' and Tg:

The samples were prepared in 100 ul cucibles and frozen in liquid nitrogen. The phase transition curves were recorded on the DSC for the 9 formulations and compared to the reference sample (R604E without additives). The samples were inserted to the DSC at -90°C.

### Result:

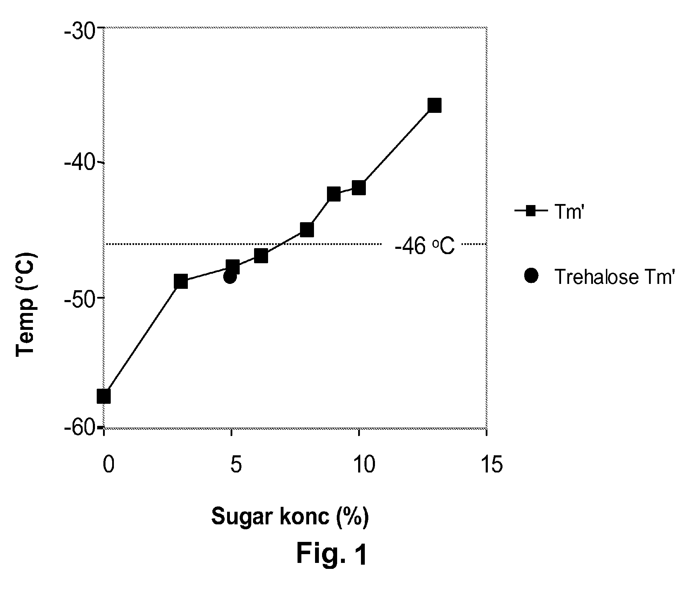

The phase transition curves can be seen in Figure 1.

The Tm' for the reference sample is found to be -54°C (onset temperature) and the Tg is measured to (-87°C) - (-92°C).

The following additives are increasing Tm':
PEG (-53°C),
Cyclodextrin (-44°C),
Maltitol (-42°C),
Trehalose (-38°C)
Fish gelatin (-37°C)
Maltodextrine 12 (-32°C)
Spray gum (-31°C)

Glycerol and sodium chloride did not increase the melting point of frozen culture pellets.

### Example 3:

In this trial the intension was to evaluate the relationship between amount of additive and the increase in Tm' measured on DSC.

### i) Material:

F-DVS CH N 19 (Batch 2421868) (commercially available frozen *LD-cultures,* Chr. Hansen A/S, Denmark).

### Manufacture of F-DVS CH N19:

The fermentation medium had the following composition: Casein peptone, 30 g/l; Primatone, 30 g/l; soy peptone, 30 g/l; yeast peptone, 15 g/l; MgSO₄, 1,5 g/l; Na-ascorbate, 3 g/l; and lactose 50 g/l.
The medium was sterilised by UHT-treatment. The finished medium had a pH of 6,5.

### Fermentation condition for F-DVS R604:

The fermentation was performed in a 100 1 fermentation tank at 30°C, stirred at 50 rpm. 1 % of the culture mentioned above was used as inoculum. The anaerobic fermentation was run with nitrogen in the headspace and a pressure of about 2 bar. The cultures were allowed to acidify to pH 6.2 . The pH was subsequently maintained at 6,2 by controlled addition of 13,4 N NH₄OH.

When no further base consumption was detected, the respective culture was cooled down to about 10°C.

Biomass was harvested and concentrated approximately x 11 on the centrifuge. The cell concentrate was divided into appropriate portions of 300 gram and formulated as specified in Table 1 below. The additives and concentrates were mixed for 30 minutes and subsequently freezed in liquid nitrogen and stored at - 50°C.

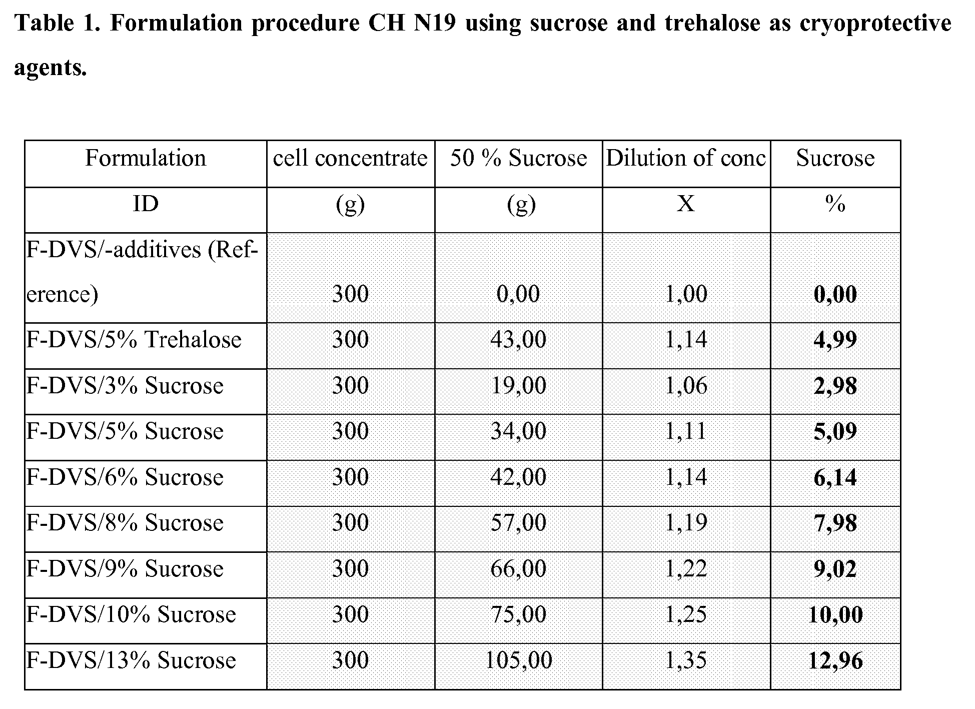

### ii) Additive solutions used for formulation to raise the melting point:

The concentration of sucrose per gram biomass varied from 3% (w/w) up to 13% (w/w). Trehalose was only tested on a 5% (w/w) level. All sucrose concentrations were prepared from a 50% (w/w) sucrose solution added to the biomass. The trehalose concentration was prepared from a 40% (w/w) solution.

### Measurement of Tm' and Tg:

The samples were prepared in 100 ul cucibles and frozen in liquid nitrogen. The phase transition curves were recorded on the DSC for the 8 formulation and compared to the the reference sample (R604E without additives). The samples were inserted to the DSC at -90°C.

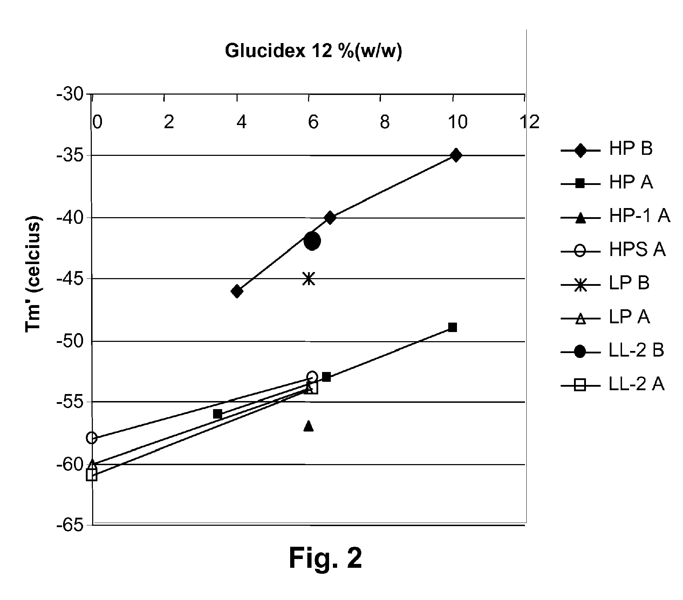

From these phase transition curves the Tm' onset and Tg onset were found. The correlation between Tm' and amount of disaccarides added can be seen in Figure 2.

From Figure 2 it can be seen that more than 3% sucrose will ensure that the frozen culture do not start melting at -50°C storage.

### REFERENCES:

EP 259739 A1, Miles Laboratories, 16 March 1988
F.J. Chavarri et al, "Cryoprotective agents for frozen concentrated starters from non-bitter Streptococcus Lactis strains", Biotechnology letters, vol 10, 1, 11- 16 (1988*)*
R. Cárcoba et al., "Influence of cryoprotectants on the viability and acidifying activity of frozen and freeze-dried cells of the novel starter strain Lactococcus lactis subsp. lactis CECT 5180", Eur Food Res Technol (2000) 211, 433 - 437
WO 00/39281, Chr. Hansen A/S, 6 July 2000
P1036 PCT application number PCT/DK2004/000025

## Claims

1. A frozen lactic acid bacteria (LAB) culture in a commercial relevant package that has a weight of at least 50 g frozen material, wherein the frozen material is present in the form of individual pellets, a content of viable bacteria of at least 10⁹ colony forming units (CFU) per g frozen material and comprises from 0.5% to 12% of an additive compound measured as w/w of the frozen material, wherein the additive compound is an additive compound that is selected from the group of compounds that,
by using an amount of 10% of the additive compound measured as w/w of the frozen material, are able to increase the Tm' (onset temperature of ice melting) of the frozen lactic acid bacteria (LAB) culture, which without the additive compound has a Tm' value from -65°C to -50°C, to a Tm' value from -49°C to -15°C (measured by DSC)
and wherein the frozen lactic acid bacteria (LAB) culture is **characterized by** that when stored at -50°C for 7 days the individual pellets of the frozen culture are not sticking together and therefore substantially remain as individual pellets where this is measured by following test
the individual pellets of the frozen culture are pellet frozen in liquid nitrogen and 100 individual pellets (around 5 - 100 g of pellets) are poured into a petridish, thus forming a thin layer of loose individual single pellets, placed at -50°C for 7 days of storage and examined to see if the pellets are still loose or if the pellets had made clumps or are sticking together wherein the criteria for that the individual pellets of the frozen culture substantially remain as individual pellets are that at least 80 of the 100 individual pellets remain as loose individual single pellets;
with the exception of a frozen lactic acid bacteria (LAB) culture that comprises LAB that are that are able to utilize sucrose and wherein the culture comprises cryoprotective agent compound selected from the group consisting of sucrose in an amount from 2 % to 12 % of sucrose measured as w/w of the frozen material; and trehalose in an amount from 4 % to 6 % of trehalose measured as w/w of the frozen material.

2. The frozen culture of claim 1, wherein the culture is a mixed mesophilic culture consisting of mesophilic bacteria having optimum growth temperatures at about 30°C.

3. The frozen culture of claim 1 or 2, wherein the LAB is a LAB selected from the group comprising *Bifidobacterium* spp., *Brevibacterium* spp., *Propionibacterium* spp., *Lactococcus* spp. including *Lactococcus lactis* subsp. *lactis* and *Lactococcus lactis* subsp. *cremoris, Lactobacillus* spp. including *Lactobacillus acidophilus, Streptococcus* spp., *Enterococcus* spp., *Pediococcus* spp., *Leuconostoc* spp., *Oenococcus* spp. and fungal spp. including *Pencillium* spp., *Cryptococcus* spp., *Debraryomyces* spp., *Klyveromyces* spp. and *Saccharomyces* spp.

4. The frozen culture of any of the preceding claims, wherein the frozen lactic acid bacteria (LAB) culture is a culture which without comprising the additive compound according to claim 1 has a Tm' value of from -65°C to -50°C.

5. The frozen culture of any of the preceding claims, wherein the frozen lactic acid bacteria culture comprises from 3% to 7% of the additive compound measured as w/w of the frozen material.

6. The frozen culture of any of the preceding claims, wherein the additive compound is an additive compound selected from the group consisting of Cyclodextrin, Maltitol, Trehalose, Fish gelatin, Maltodextrine and Spray gum.

7. A method for making a frozen lactic acid bacteria (LAB) culture of any of the claims 1 to 6 comprising following steps:
(i) adding an additive compound to viable bacteria to get at least 50 g of material with a content of viable bacteria of at least 10⁹ colony forming units (CFU) per g material and comprising the additive compound in an amount from 0.5% to 12% measured as w/w of the material,
(ii) freezing the material to get frozen material, and
(iii) packing the frozen material in a suitable way to get a packed frozen lactic acid bacteria (LAB) culture of any of the claims 1 to 6.

8. The method of claim 7, wherein
before making the addition of the additive compound according to step (i) of claim 7 one has measured the Tm' value of the frozen lactic acid bacteria (LAB) culture without comprising the additive compound and identified that it has a Tm' value of from -65°C to -50°C;
and
after the addition of the additive compound is the Tm' value of the frozen lactic acid bacteria (LAB) culture comprising the additive compound measured and it is verified that the Tm' value is from -49°C to -15°C, more preferably from -39°C to -15°C.

9. A frozen lactic acid bacteria (LAB) culture obtainable by the method for making a frozen lactic acid bacteria (LAB) culture of claim 7 or 8.

10. Use of the frozen lactic acid bacteria (LAB) culture of any of claims 1-6 and 9 in a process for making a food or feed product.
